# EUROPÄISCHE PATENTSCHRIFT

(11) **EP 1 641 513 B1**
(45) Veröffentlichungstag und Bekanntmachung des Hinweises auf die Patenterteilung: **17.06.2009**
(21) Anmeldenummer: 04738722.0
(22) Anmeldetag: 18.06.2004
(51) Int. Cl.: A61M 25/06, A61M 25/01

(54) **KATHETERAPPLIKATIONSSYSTEM**
CATHETER APPLICATION SYSTEM
SYSTEME DE MISE EN PLACE DE CATHETER

(30) Priorität: 27.06.2003 DE 10329126
(43) Veröffentlichungstag der Anmeldung: 05.04.2006
(73) Patentinhaber: Willy Rüsch GmbH, 71394 Kernen-Rommelshausen (DE)
(72) Erfinder: SASSER, Ross, Cumming, GA 30040 (US); GIEBMEYER, Carsten, 76774 Leimersheim (DE)
(74) Vertreter: Grünecker, Kinkeldey, Stockmair & Schwanhäusser Anwaltssozietät
(86) Internationale Anmeldenummer: PCT/DE2004/001272
(87) Internationale Veröffentlichungsnummer: WO 2005/004970

(56) Entgegenhaltungen:
- DE-A- 2 456 980
- DE-U- 9 001 146
- GB-A- 2 031 733
- US-A- 3 421 509
- US-A- 3 683 928
- US-A- 5 417 666
- US-A- 6 004 305

## Beschreibung

Die Erfindung betrifft ein Katheterapplikationssystem, insbesondere zum Einführen eines Blasenkatheters in die Harnröhre (Urethra). Das Kathetersystem verfügt über eine Einführhülse zum Erleichtern des Einführens des Katheters, wobei der Katheter möglichst steril eingeführt wird, insbesondere ohne dass Mikroorganismen aus dem vorderen Bereich der Harnröhre bis zur Blase eingeschleppt werden.
Katheter werden für Diagnose- und Therapiezwecke, z.B. zur suprapubischen Blasendrainage oder zur Harnableitung über die Urethra verwendet. Die Katheter werden vom Hersteller steril verpackt für deren Anwendung (Applikation) zur Verfügung gestellt. Vor der Applikation eines Katheters wird der Bereich um die Körperöffnung, in welche der Katheter eingeführt werden soll, gewaschen und desinfiziert. Eine absolut sichere Desinfektion, insbesondere der Harnröhrenmündung (Orificium) und des vorderen Teiles der Harnröhre ist indes praktisch nicht durchzuführen. Insbesondere im vorderen Teil der Harnröhre, welcher in das Orificium übergeht, verbleibt immer eine mikrobielle Kontamination, so dass der sterile Katheter beim Einführen in die Harnröhre einen nicht sterilen Bereich passieren muss. Das Verschleppen der Mikroorganismen aus diesem Harnröhrenbereich in die Blase oder in hintere Harnröhrenbereiche muss möglichst vermieden werden. Insbesondere bei Langzeitkathetern, welche über mehrere Tage appliziert bleiben, müssen daher beim Einführen besondere Vorsichtsmaßnahmen getroffen werden.

Beim Katherismus ist es zur Reduzierung der Gleitreibung bekannt, dass die Außenoberfläche des Katheters mit einer Flüssigkeit, z.B. einem Gleitmittel oder einer wässrigen Kochsalzlösung, benetzt wird, damit der Katheter möglichst schmerzfrei und ohne weitere Irritationen hervorzurufen in ein Hohlorgan des menschlichen Körpers eingeführt werden kann. Dies kann auch durch andere Beschichtungen zur Verbesserung der Gleiteigenschaften erfolgen. Weiterhin werden Blasenkatheter häufig verwendet, die hierzu eine hydrophile Außenoberfläche über deren Schaftlänge aufweisen, welche vor der Applikation des Katheters mittels einer wasserhaltigen Flüssigkeit aktiviert werden. Bei einer hydrophilen Oberfläche binden sich, bei einer Benetzung der Oberfläche mit Wasser, Wassermoleküle an die Oberfläche. Dadurch entsteht eine sanfte, glatte Oberfläche, welche ein komfortables Einführen des Katheters ermöglicht. Hierbei handelt es sich um Ausführungsformen, welche sich zur Vermeidung von Beschwerden beim Einführen eines Katheters und bei dessen Applikation in der Vergangenheit bewährt haben.

Weiter ist es bekannt, sowohl die Innenoberfläche als auch die Außenoberfläche mit einer antimikrobiellen Beschichtung zur Reduzierung der Bakterienadhärenz, z.B. aus Silber, zu versehen.

Die DT 24 56 980 A1, die US 3,421,509 und die US 4,652,259 offenbaren ein Katheterapplikationssystem mit einer Einführhülse für Harnröhrenkatheter, die in den vorderen Bereich des mit Bakterien infizierten Abschnitts der Harnröhre eingeführt wird, bis ein Anschlagelement, z.B. ein Ring, das Einschieben der Einführhülse in die Harnröhre begrenzt. Nachdem die Einführhülse in die Harnröhre eingeführt worden ist, kann ein Katheter durch die Einführhülse hindurchgeschoben werden und dieser Katheter öffnet einen Schlitz am distalen Ende der Einführhülse und kann anschließend bis in die Blase vorgeschoben werden. Durch die Einführhilfe wird der Transport von Mikroorganismen innerhalb der terminalen Urethra auf eine kurze Distanz beschränkt und für den einzuführenden Katheter eine sterile Passage durch den mikrobiell kontaminierten Bereich der Harnröhre geschaffen. DE 9001146 U zeigt eine trennbare Einführhülse für Katheter.

US 6004305 offenbart die Merkmale des Oberbegriffs von Anspruch 1.

Bei den Katheterapplikationssystemen zur intermittierenden Katheterisierung gemäß dem Stand der Technik ist es vorgesehen, dass die Einführhilfe nach Applikation des Katheters am Katheter verbleibt, da die Einführhilfe den Katheterschaft des Katheters vollständig nach Art eines Ringes umschließt.

Der Erfindung liegt die Aufgabe zugrunde, ein Katheterapplikationssystem bereitzustellen, welches die Nachteile des Standes der Technik vermeidet und insbesondere einfach, schnell und kostengünstig anzuwenden ist, wobei ein Beitrag zum Vermeiden oder Reduzieren von katheterassoziierten Infekten, insbesondere Harnwegsinfekten, geleistet werden soll.

Diese Aufgabe wird durch die Vorrichtung des unabhängigen Anspruches gelöst. Die abhängigen Ansprüche stellen bevorzugte Ausführungsformen der Erfindung dar.

Erfindungsgemäß wird die Aufgabe dadurch gelöst, dass ein Katheterapplikationssystem mit einem, bevorzugt dauerhaft zur Blasendrainage applizierbaren, Katheter (Blasenkatheter), der eine Katheterspitze aufweist, einer hohlen Umhüllung, in der der Katheter gleitbar angeordnet ist und einer Einführhilfe am distalen Ende der Umhüllung zur Verfügung gestellt wird. Die Umhüllung gewährleistet zusätzlich eine primär sterile Verpackung des Katheters und der innerhalb der Umhüllung befindlichen Einführhilfenteile. Die Einführhilfe ist zur Durchführung des Katheters durch dieselbe eingerichtet, wobei die Einführhilfe Einführhilfenteile, nämlich eine Einführhülse mit einem Verschlusselement, das für die Durchführung des Katheters geöffnet werden kann, und ein sich bevorzugt ausserhalb der Umhüllung erstreckendes Anschlagelement zur Begrenzung einer Einführung der Einführhülse in eine Körperöffnung aufweist. Das Verschlusselement ist dabei bevorzugt integraler Bestandteil der Einführhülse. Die Einfübrhülse und/oder das Anschlagelement sind dergestalt als splittbare Einführhilfenteile ausgeführt, dass diese bei durchgeführtem Katheter vom Katheter entfernbar sind. Die splittbaren Einfübrhilfenteile sind also teilbar ausgeführt. Dadurch sind diese Einführhilfenteile vom Katheter abnehmbar. Der Katheter wird bei dessen Applikation durch die Einführhilfe und damit durch deren Einführhilfenteile durchgeführt. Die Einführhilfenteile haben also eine Durchführungsöffnung. Nachdem der Katheter durch die Einführhilfe durchgeführt wurde umschließen die Einführhilfenteile den Katheter damit ringartig. Unter splittbar ist zu verstehen, dass die derart ausgeführten Einführhilfenteile so in Einzelteile zerlegbar sind, dass sie nach der Zerlegung den Katheter nicht mehr umfassen. Die splittbare Ausführung hat den Vorteil, dass die Einführhilfe problemlos vom Katheter entfernt werden kann, ohne dass der Katheter vollständig durch die Einführhilfe durchgeführt werden muss, was z.B. im Falle eines fest an den Katheter angebrachten Urinbeutels nicht möglich wäre. Damit kann die nach Applikation des Katheters mikrobiell kontaminierte Einführungshilfe, bzw. zumindest die kontaminierte Einführhülse, leicht vom Katheter entfernt werden, wodurch einer aufsteigenden Infektion entgegengewirkt werden kann.

Zur Applikation des Blasenkatheters wird, nach dem Bereitstellen des erfindungsgemäßen Katheterapplikationssystems, die Einführhülse, die von innen her durch die Katheterspitze gestützt wird, in den an das Orificium angrenzenden vorderen Bereich der Harnröhre des Patienten so weit eingeführt bis das Anschlagelement ein weiteres Einführen verhindert. Danach wird der Katheter mit der Katheterspitze voran durch die Einführhilfe durchgeführt, wodurch das Verschlusselement mit der Katheterspitze eröffnet wird. Daraufhin wird der Katheter durch die Einführhilfe vorgeschoben bis die Katheterspitze in die Blase des Patienten vorgedrungen ist. Nach Blocken des Rückhaltesystems (Ballon) des Katheters wird die Einführhülse aus der Harnröhre des Patienten herausgezogen und die Einführhülse, bevorzugt die gesamte Einführhilfe, zerteilt (gesplittet), um diese von dem Katheter abzunehmen.

In einer anderen Ausführungsform weist das Verschlusselement des Katheterapplikationssystems eine Perforierung oder einen Kreuzschlitz auf, so dass die Öffnung bei der Durchführung des Katheters durch ein Durchstoßen der Perforierung, bzw. des Kreuzschlitzes ermöglicht ist. Die Ausführung des Verschlusselements mit einer Perforierung oder einem Kreuzschlitz hat den Vorteil, dass keine zusätzlichen Teile zur Gewährleistung der Durchstoßbarkeit des Verschlusselements notwendig sind und das Verschlusselement seine primär geschlossene Spitzenform behält.

Die splittbar ausgeführten Einfuhrhilfenteile bestehen bevorzugt jeweils aus mindestens zwei Segmenten, die zusammengefügt den durchgeführten Katheter ringartig umfassen. Die Segmente haben also jeweils einen an die Durchführungsöffnung angrenzenden Bereich. Im einfachsten Fall bestehen die splittbaren Einführungshilfeteile aus zwei symmetrischen Segmenten. Ist z.B. das Anschlagelement als Ring ausgebildet, so haben dessen zwei Segmente die Form eines halben Rings. Die segmentierte Ausuhrungsform hat den Vorteil, dass die Splittbarkeit mittels einer einfachen Aufteilung der Einfiihrungshilfeteile gewährleistet wird. Diese Ausführungsform ist daher einfach und kostengünstig herzustellen.

Die Segmente der splittbar ausgeführten Einführhilfenteile werden mittels Sollbruchstellen, insbesondere Perforationen und/oder Klebeband und/oder Kreppband zusammengehalten. Besonders vorteilhaft ist dabei eine Ausführung mit ineinander verrastenden Elementen. Dadurch werden für die erfindungsgemäße Splittbarkeit der Einführhilfe des Katheterapplikationssystems keine zusätzlichen Teile benötigt. Es entstehen keine zusätzlichen Produktionskosten. Auch die Applikation des Katheters wird nicht gegenüber eines Katheters nach Stand der Technik erschwert.

Bevorzugt ist die Einführhilfe des Katheterapplikationssystems, insbesondere das Anschlagelement, als Handgriff ausgeführt, wobei bevorzugt eine Durchgangsöffnung im Handgriff vorhanden ist. Die Durchgangsöffnung kann entweder nur einseitig oder beidseitig ausgeführt sein. Das Anschlagelement kann dazu aus mehreren Teilen zusammengefügt sein.

Mittels einer beidseitigen Duchgangsöffnung im Handgriff kann der Katheter beim Durchführen durch die Einführhilfe z.B. zwischen zwei Fingern in der Durchgangsöffnung erfasst werden, wodurch die Applikation des Katheters erleichtert wird. Bei einer einseitigen Durchgangsöffnung kann der Katheter innerhalb des Handgriffs, z.B. mit einem Finger der den Katheter applizierenden Person an eine Innenseite des Handgriffes gedrückt und damit festgehalten werden.

Die Einführhilfe weist erfindungsgemäß eine abnehmbare Schutzkappe über der Einführhülse auf. Dies gewährleistet einen Schutz der Einführhülse vor mechanischer Beschädigung und hält die Einführhülse steril. Die Schutzkappe kann auch noch die Oberfläche des Anschlagelements mit abdecken.

Der Katheter weist bevorzugt eine hydrophobe/oleophobe Außenoberfläche und/oder auf dessen Außen- und/oder Innenoberfläche eine antimikrobielle, bevorzugt eine Silberbeschichtung, auf.

Die Schutzkappe und/oder die Einführhülse sind bevorzugt mit jeweils einer Flüssigkeit befüllt. Weiter kann ein mit einer Flüssigkeit befülltes Flüssigkeitsreservoir, von dem Katheter bei dessen Applikation durchstoßbar, in der Einführhilfe angeordnet sein. Diese Ausführungsformen gewährleisten eine Flüssigkeitsbenetzung des Katheters beim Einführen in eine Körperöffnung. Dadurch wird ein weitgehend schmerzfreies Einführen ermöglicht. Das Flüssigkeitsreservoir gewährleistet eine ausreichende Dosierung der Flüssigkeit entsprechend des persönlichen Bedarfs des Anwenders. Eine schonende Katheterisierung ohne zusätzliche Hilfsmittel und Kathetervorbereitungen wird ermöglicht.

Bei den Flüssigkeiten handelt es sich bevorzugt um ein Gleitgel und/oder Wasser und/oder physiologische Kochsalzlösung (0,9 gewichtsprozentige Lösung).

An dem Katheter des Katheterapplikationssystems ist bevorzugt ein vorkonnektiertes Auffangbehältnis, z.B. ein Urinbeutel befestigt. Dadurch wird bei der Anwendung des Katheterapplikationssystems vermieden, dass beim Verbinden des Katheters mit einem Auffangbehältnis eine mikrobielle Kontamination der Katheterinnenoberfläche stattfinden kann.

Insbesondere im Falle eines dauerhaften Blasenkatheters weist der Katheter im Bereich seiner Katheterspitze ein aktivierbares Rückhaltesystem, z.B. einen aufblasbaren Ballon (Cuff) auf, wobei der Ballon im aufgeblasenen Zustand eine Arretierung der Katheterspitze in der Blase des Patienten bewirkt. Dazu ist im Bereich der Katheterspitze ein Hohlraum vorgesehen, welcher über eine, entlang des Katheters geführte schlauchartige Leitung mit einem Fluid befüllbar ist. Eine sichere Platzierung des Dauerkatheters in der Blase kann damit gewährleistet werden.

Insbesondere für einen Blasenkatheter weist die Einführhülse eine Hülsenlänge zwischen 1,55 und 2 cm und einen Durchmesser, der dem jeweiligen Außendurchmesser des verwendeten Dauerkatheters angepasst ist auf. Diese Dimensionierungen entsprechen der Dimension der Harnröhre, insbesondere der Länge des natürlich mikrobiell kontaminierten Bereichs derselben.

Bevorzugt ist eine Arretiervorrichtung, die das Verschieben des Katheters durch die Einführhilfe nur in Einführrichtung ermöglicht, innerhalb der Einführhilfe des Katheterapplikationssystems vorgesehen. Eine derartige Arretiervorrichtung vermeidet, dass der Katheter unbeabsichtigt während des Einführens wieder aus z.B. der Urethra hinausgleitet, was sehr leicht zu dessen Verunreinigung führen würde.

Weitere Vorteile ergeben sich aus der Beschreibung und den beigefügten Zeichnungen. Die vorstehend genannten und die noch weiter aufgeführten Merkmale der Erfindung können jeweils einzeln oder in Kombination miteinander verwendet werden. Die erwähnten Ausführungsformen sind nicht als abschließende Aufzählung zu verstehen, sondern haben vielmehr beispielhaften Charakter.

Die Erfindung wird nachfolgend anhand von Ausführungsbeispielen unter Bezugnahme auf die Zeichnungen näher erläutert.
Fig. 1 zeigt ein erfindungsgemäßes Katheterapplikationssystem;
Fig. 2a zeigt eine Explosionszeichnung der Einführhilfe eines erfindungsgemäßen Katheterapplikationssystems; und
Fig. 2b zeigt die in Fig. 2a dargestellte Einführhilfe in zusammengebautem Zustand.
Fig. 3 zeigt eine Ausführungsform eines Anschlagelementes mit einer einseitigen Durchgangsöffnung.

Die Fign. der Zeichnungen zeigen den erfindungsgemäßen Gegenstand stark schematisiert und sind nicht maßstäblich zu verstehen. Die einzelnen Bestandteile des erfindungsgemäßen Gegenstandes sind so dargestellt, dass ihr Aufbau gut gezeigt werden kann.

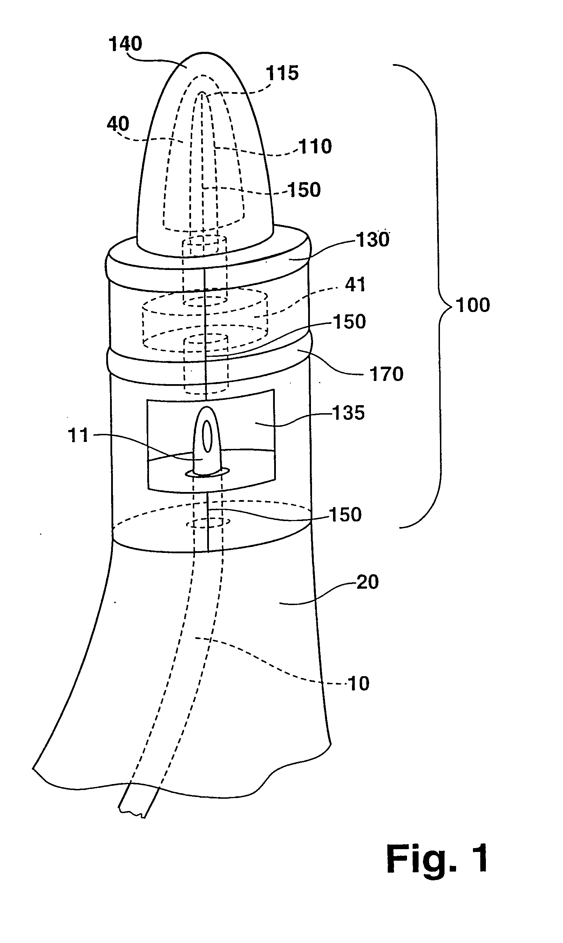

In Fig. 1 ist das erfindungsgemäße Katheterapplikationssystem dargestellt. Das Gesamtsystem besteht aus einem geschlossenen sterilen Kathetersystem mit einem Katheter 10 aus Silikon oder PVC oder Polyurethan (PU) oder Latex, welches eine sterile Katheterisierung ermöglicht. Dies dient zur Vorbeugung von katheterinduzierten Harnwegsinfektionen. Dazu tragen das geschlossene sterile System, eine Silberbeschichtung und eine hydrophobe/oleophobe Beschichtung sowohl der Außen- als auch der Innenoberfläche des Katheters 10 bei. Die Beschichtung erfolgt mittels einer Nanobeschichtungstechnologie. Der Katheter 10 ist bevorzugt dauerhaft zur Blasendrainage applizierbar. Der Katheter 10 hat eine Katheterspitze 11 mit mindestens einer Drainageöffnung (Auge), welche zur Verbesserung der Gleiteigenschaften des Katheters mit abgerundeten Kanten versehen ist. Der Katheter 10 ist innerhalb einer hohlen Umhüllung 20 gleitbar angeordnet. Diese Innenverpackung besteht aus Kunststofffolie. Am distalen Ende der Umhüllung 20 befindet sich eine Einführhilfe 100, durch die der Katheter durchgeführt werden kann. Die Umhüllung 20 besteht aus einem Kunststoffbeutel (Polyethylen-Beutel), der an der Einführhilfe, z.B mittels Klebeband oder eines Schrumpfschlauches befestigt ist. Dieser Kunststoffbeutel umfasst die restlichen Bauteile des Katheterapplikationssystems primär steril. Die Einführhilfe ist aus Einführhilfenteilen, nämlich einer Einführhülse 110 mit einem Verschlusselement 115 und einem Anschlagelement 130 zur Begrenzung einer Einführung der Einführhülse in eine Körperöffnung zusammengefügt. Das Verschlusselement 115 kann für die Durchführung des Katheters geöffnet werden. Dies erfolgt bevorzugt durch Durchstoßen des Verschlusselements 115 mit der Katheterspitze 11. Das Verschlusselement 115 ist dazu, z.B. als eine sollbruchstellenartige Perforierung der Einführhülse 110 oder als Kreuzschlitz in der Einführhülse ausgeführt. Die Einführhülse (Einführmembran) ist aus weichem Kunststoff gefertigt. Das Anschlagelement 130 erstreckt sich außerhalb der Umhüllung 20. Die Einführhülse 110 und das Anschlagelement 130 sind splittbar ausgestaltet, d. h., sie sind in mindestens zwei segmentartige Teile aufteilbar, wodurch sie bei durchgeführtem Katheter vom Katheter entfernbar sind. Dazu ist sowohl im Anschlagelement 130, als auch in der Einführhülse 110 eine Sollbruchstelle 150 vorgesehen. Die teilbare Spitze, also die Einführhülse 110 dient als Einführhilfe für den Katheter mit zusätzlicher Überbrückung der ersten Zentimeter der Harnröhre. Das Anschlagelement 130 ist derart ausgeführt, dass es als Griff zum Greifen mit einer Hand dienen kann. Dazu ist das Anschlagelement auf seiner von der Einführhülse 110 abgewandten Seite verlängert ausgeführt. Innerhalb dieser verlängerten Ausführung sind ein Flüssigkeitsreservoir 41 und eine Durchgangsöffnung 135 angeordnet. Der Katheter wird derart durch das Anschlagelement 130 durchgeführt, dass er z. B. mit zwei Fingern durch die Durchgangsöffnung 135 ergriffen werden kann und dass der Katheter das Flüssigkeitsreservoir 41 durchstößt. Der Basiskörper der Einführhilfe, d.h. das Anschlagelement ist aus hartem Kunststoff gefertigt. Ein Halteband mit Klettverschluss (Kreppband) 170 sichert den Zusammenhalt der segmentartigen Teile (Segmente) des Anschlagelements 130 und hält gleichzeitig die Umhüllung 20 fest an dem Anschlagelement 130. Dies kann auch durch einen Schrumpfschlauch gewährleistet sein. Die Einführhülse 110 ist mit einer Schutzkappe 140, die sich an deren Basis verbreitert und damit auch das Anschlagelement zumindest teilweise überdeckt, versehen. Der von der Schutzkappe gebildete Hohlraum 40 über der Einführhülse 110 ist mit einer Flüssigkeit, z.B. einem wasserbasierten Gleitgel, gefüllt. Die Einführhülse 110 ist bevorzugt ebenfalls mit Gleitgel vorab befüllt. Dadurch befindet sich eine ausreichende Menge des Gleitgels auf dem Katheter und auf der Einführspitze, d.h. der Einführhülse. Die Schutzkappe 140 ist aus hartem Kunststoff gefertigt und ist leicht durch einfaches Drehen oder Abziehen abnehmbar.
Am von der Katheterspitze abgewandten Ende des Katheters weist dieser üblicherweise einen Trichter auf. Diese Trichter ist herstellerseits an ein Urinableitesystem angebunden (preconnected). Z.B. ist ein Auffangbehältnis, z.B. ein Urinbeutel befestigt. Die Verbindung vom Urinbeutel zum Katheter ist bevorzugt mittels eines Schrumpfschlauches gesichert. Die Umhüllung des Katheterapplikationssystems endet am Trichter des Katheters.
Das Katheterapplikationssystem kann weiter einen Urinrücklaufschutz und/oder einen am Urinbeutel befestigten oder integrierten Henkel oder ein Loch zum Halten und Aufhängen des Beutels aufweisen. Üblicherweise verfügen dauerhaft applizierbare Blasenkatheter weiterhin über eine Urinentnahmestelle an der Konnektionsstelle des Urinbeutels zum Katheter, eine pasteursche Tropfkammer zur Flußunterbrechung, ein Antirefluxventil, eine Entlüftung mit antimokrobieller Funktion und/oder einen tropffreien Auslaß.
Das Katheterapplikationssystem wird in einer sterilen Außenverpackung bereit gestellt. Diese Aussenverpackung ist so aufgebaut, dass sie zur Applikation des Katheters von der Einführhilfe in Richtung Urinbeutel zurückgeschoben (Peel-Back) werden kann.

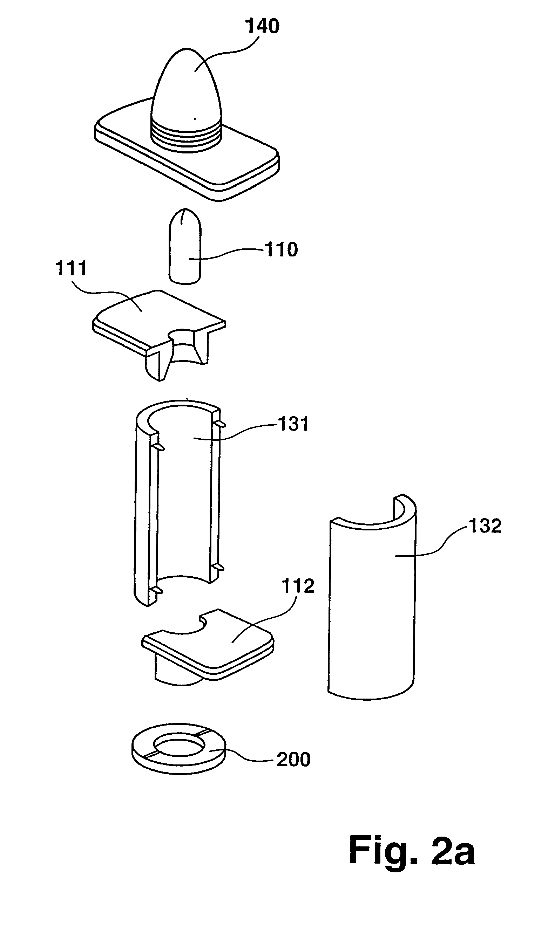

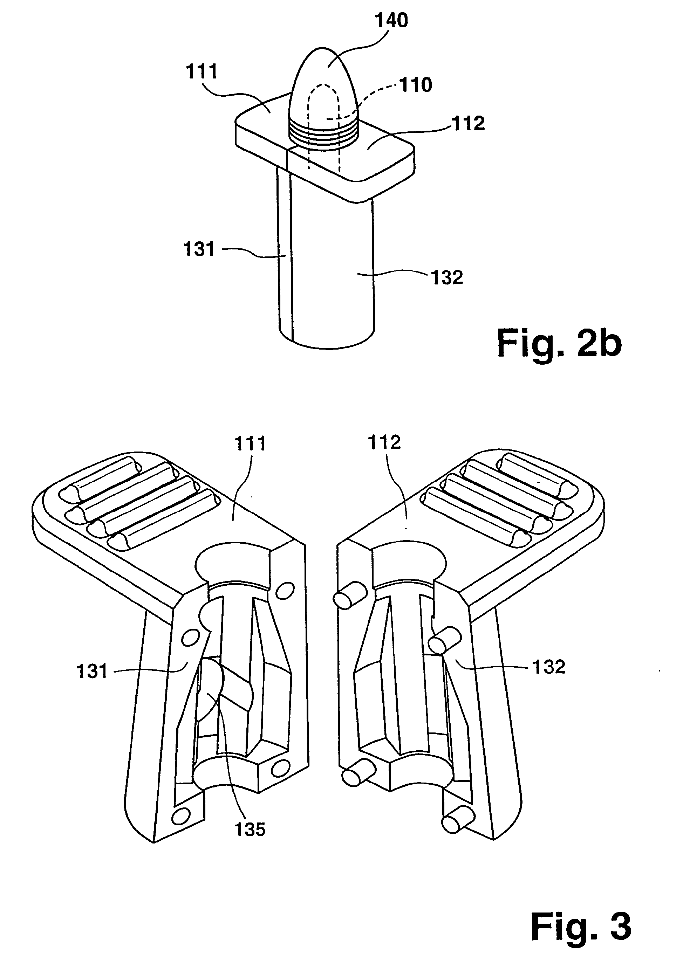

In Fig. 2 wird eine Einführhilfe eines erfindungsgemäßen Katheterapplikationssystems dargestellt. Fig. 2a zeigt die Einführhilfe in einer Explosionszeichnung und Fig. 2b zeigt die Einführhilfe im zusammengebauten Zustand. Dargestellt sind eine Schutzkappe 140, eine Einführhülse 110 und ein splittbar ausgeführtes Anschlagelement. Das Anschlagelement ist so ausgeführt, dass es die Dimension eines Griffs aufweist. Hierzu besteht das Anschlagelement aus mehreren Teilen. Dies sind zwei Flügelsegmente 111, 112 und zwei Griffsegmente 131, 132 sowie ein Abschlussring 200. Die Flügelsegmente ergeben zusammen eine Fläche, welche den Anschlag an die Penisspitze bilden.

In Fig. 3 ist eine Ausführungsform eines Anschlagelementes mit einer einseitigen Durchgangsöffnung 135 dargestellt. Das Anschlagelement besteht aus zwei Flügelsegmenten 111, 112 und zwei Griffsegmenten 131, 132. Insgesamt ist das Anschlagelement aus zwei Teilen gefertigt, welche in der Figur auseinander gesplittet dargestellt sind. Die Oberfläche des Anschlagelements, welche zur Begrenzung des Einführens der Einführhilfe beim Einführen den Körper des Patienten beführt, weist eine Riffelung auf. Die beiden Teile weisen Verrastungen auf, mittels derer sie splittbar verbindbar sind. Die Verrastungen bestehen aus zapfenartig geformten Vorsprüngen in einem Teil, die auf der entsprechend gegenüber liegenden Seite des anderen Teils in zu der Zapfenform passende Löcher einrastbar sind. Im zusammengefügten Zustand bilden die Teile einen Hohlraum. Innerhalb dieses Hohlraumes sind Stege angeordnet, welche eine exakte Führung eines durchzuführenden Katheters gewährleisten. Ein erstes Teil ist aus einem Griffsegment 131 und einem Flügelsegment 111 einstückig gebildet. Dieses erste Teil weist eine Durchgangsöffnung 135 auf. In diesem ersten Teil sind die Löcher von vier Verrastungen vorhanden. Das zweite Teil ist ebenfalls aus einem Griffsegment 132 und einem Flügelsegment 112 einstückig gebildet. Es weist jedoch keine Durchgangsöffnung auf. Die Durchgangsöffnung ist somit nur einseitig ausgebildet. Weiter weist das zweite Teil die Zapfen der vier Verrastungen auf.

Erfindungsgemäß sind zumindest die Griffsegmente 131, 132 aus einem weichen, deformierbaren, mit bevorzugt elastischen Eigenschaften, Material ausgebildet. Mit den Fingern eines Anwenders lassen sich derartige Griffsegmente ohne größeren Kraftaufwand so stark deformieren, dass ein durch das Anschlagelement hindurchgeführter Katheter in jeder Lage fixiert gehalten werden kann. Ein ungewolltes Verrutschen eines Katheters im Anschlagelement ist nicht mehr möglich und auf eine Durchgangsöffnung, wie in Fig. 1 und Fig. 3 gezeigt, kann verzichtet werden.

### Bezugszeichenliste

- 10: Katheter
- 11: Katheterspitze
- 20: Umhüllung
- 40: Hohlraum
- 41: Flüssigkeitsreservoir
- 100: Einführhilfe
- 110: Einführhülse
- 111, 112: Flügelsegment
- 115: Verschlusselement
- 130: Anschlagelement
- 131, 132: Griffsegment
- 135: Durchgangsöffnung
- 140: Schutzkappe
- 150: Sollbruchstelle
- 170: Klettverschluss
- 200: Abschlußring

## Patentansprüche

1. Katheterapplikationssystem mit
einem dauerhaft zur Blasendrainage applizierbaren Katheter (10), der eine Katheterspitze (11) und einen im Bereich der Katheterspitze (11) angeordneten aufblasbaren Ballon aufweist, wobei der Ballon im aufgeblasenen Zustand eine Arretierung der Katheterspitze (11), bevorzugt in der Blase des Patienten, bewirkt,
einer hohlen Umhüllung (20), in der der Katheter gleitbar angeordnet ist und einer Einführhilfe (100) am distalen Ende der Umhüllung, eingerichtet zur Durchführung des Katheters (10) durch dieselbe, wobei die Einführhilfe Einführhilfenteile, nämlich
eine Einführhülse (110) mit einem Verschlusselement (115), das für die Durchführung des Katheters (10) geöffnet werden kann, und
ein sich ausserhalb der Umhüllung erstreckendes Anschlagelement (130), zur Begrenzung einer Einführung der Einführhülse (110) in eine Körperöffnung, aufweist, **dadurch gekennzeichnet, dass** die Einführhilfe eine abnehmbare Schutzkappe (140) über der Einführhülse (110) aufweist, und dass die Einführhülse (110) und/oder das Anschlagelement (130) dergestalt als splittbare Einführhilfenteile ausgeführt sind, dass diese bei durchgeführtem Katheter (10) vom Katheter (10) entfernbar sind, und, dass das Anschlagelement Griffsegmente aufweist, wobei die Griffsegmente aus einem weichen, deformierbaren Material, mit bevorzugt elastischen Eigenschaften ausgebildet sind.

2. Katheterapplikationssystem nach Anspruch 1, **dadurch gekennzeichnet, dass** das Verschlusselement (115) eine Perforierung derart aufweist, dass die Öffnung bei der Durchführung des Katheters (10) durch ein Durchstoßen der Perforierung ermöglicht ist oder das Verschlusselement (115) einen Kreuzschlitz derart aufweist, dass die Öffnung bei der Durchführung des Katheters (10) durch ein Durchstoßen des Kreuzschlitzes ermöglicht.

3. Katheterapplikationssystem nach mindestens einem der Ansprüche 1 bis 2, **dadurch gekennzeichnet, dass** die splittbar ausgeführten Einführhilfenteile jeweils aus mindestens zwei Segmenten (111, 112, 131, 132) bestehen, die zusammengefügt den durchgeführten Katheter (10) ringartig umfassen.

4. Katheterapplikationssystem nach Anspruch 3, **dadurch gekennzeichnet, dass** die Segmente (111, 112, 131, 132) der splittbar ausgeführten Einführhilfenteile mittels Sollbruchstellen (150), insbesondere Perforationen und/oder Klebeband und/oder Kreppband, und/oder Verrastelementen zusammengehalten werden.

5. Katheterapplikationssystem nach mindestens einem der Ansprüche 1 bis 4, **dadurch gekennzeichnet, dass** die Einführhilfe, insbesondere das Anschlagelement (130), als handgriffgroßes Teil ausgeführt ist, wobei bevorzugt eine einseitige oder doppelseitige Durchgangsöffnung (135) in dem Teil ausgebildet ist.

6. Katheterapplikationssystem nach mindestens einem der Ansprüche 1 bis 5, **dadurch gekennzeichnet, dass** der Katheter (10) eine hydrophobe/oleophobe Außenoberfläche und/oder auf dessen Außen- und/oder Innenoberfläche eine antimikrobielle, bevorzugt eine Silberbeschichtung, aufweist.

7. Katheterapplikationssystem nach mindestens einem der Ansprüche 1 bis 6, **dadurch gekennzeichnet, dass** die Schutzkappe (140) und/oder die Einführhülse (110) mit jeweils einer Flüssigkeit befüllt sind und/oder dass ein mit einer Flüssigkeit befülltes Flüssigkeitsreservoir (41), von dem Katheter (10) bei dessen Applikation durchstoßbar, in der Einführhilfe (10) angeordnet ist.

8. Katheterapplikationssystem nach Anspruch 7, **dadurch gekennzeichnet, dass** es sich bei den Flüssigkeiten um ein Gleitgel und/oder Wasser und/oder physiologische Kochsalzlösung handelt.

9. Katheterapplikationssystem nach mindestens einem der Ansprüche 1 bis 8, **dadurch gekennzeichnet, dass** an dem Katheter (10) ein vorkonnektiertes Auffangbehältnis befestigt ist.

10. Katheterapplikationssystem nach mindestens einem der Ansprüche 1 bis 9, **dadurch gekennzeichnet, dass** die Einführhülse (110) eine Hülsenlänge zwischen 1,55 und 2 cm aufweist.

11. Katheterapplikationssystem nach mindestens einem der Ansprüche 1 bis 10, **dadurch gekennzeichnet, dass** eine Arretiervorrichtung, die das Verschieben des Katheters durch die Einführhilfe nur in Einführrichtung ermöglicht, innerhalb der Einführhilfe vorgesehen ist.

## Claims

1. A catheter application system comprising
a catheter (10) applicable for long-term bladder drainage and comprising a catheter tip (11) and an inflatable balloon arranged in the area of the catheter tip (11), the balloon in the inflated state effecting a locking of the catheter tip (11), preferably in the patient's bladder,
a hollow sheath (20) in which the catheter is slidably disposed, and an insertion aid (100) at the distal end of said sheath provided for passage of the catheter (10) through it, the insertion aid comprising insertion aid components, namely
an insertion sleeve (110) with a sealing element (115) which can be opened for passage of the catheter (10) and
a stop element (130) extending outside of said sheath for limiting insertion of the insertion sleeve (110) into a body orifice,
**characterized in that** the insertion aid comprises a removable protective cover (140) over the insertion sleeve (110), and that the insertion sleeve (110) and/or the stop element (130) are formed as splittable insertion aid components in such a way that said insertion aid components are removable from the catheter (10) after its passage, and that the stop element comprises handle segments, said handle segments being made of a soft deformable material with preferably resilient characteristics.

2. The catheter application system according to claim 1, **characterized in that** the sealing element (115) comprises a perforation formed in such a way that a penetration of said perforation enables the opening during the passage of the catheter (10), or that the sealing element (115) comprises a cross-shaped slit in such a way that a penetration of said cross-shaped slit enables the opening during the passage of the catheter (10).

3. The catheter application system according to at least one of the claims 1 to 2, **characterized in that** the splittable insertion aid components each consist of at least two segments (111, 112, 131, 132), which, in the assembled state, encompass the catheter (10) after its passage like a ring.

4. The catheter application system according to claim 3, **characterized in that** the segments (111, 112, 131, 132) of the splittable insertion aid components are held together by predetermined breaking points (150), in particular perforations and/or adhesive tape and/or crepe tape and/or locking elements.

5. The catheter application system according to at least one of the claims 1 to 4, **characterized in that** the insertion aid, in particular the stop element (130), is formed as a part having the size of a handle, wherein a preferably unilateral or bilateral through hole (135) is formed within said part.

6. The catheter application system according to at least one of the claims 1 to 5, **characterized in that** the catheter (10) comprises a hydrophobic/oleophobic outer surface and/or an antimicrobial, preferably silver coating on its outer and/or inner surfaces.

7. The catheter application system according to at least one of the claims 1 to 6, **characterized in that** the protective cover (140) and/or the insertion sleeve (110) are filled with a liquid each, and/or a liquid reservoir (41) filled with a liquid is disposed in the insertion aid (10), said liquid reservoir (41) being penetratable by the catheter (10) during its application.

8. The catheter application system according to claim 7, **characterized in that** the liquids are a lubricant gel and/or water and/or physiological saline solution.

9. The catheter application system according to at least one of the claims 1 to 8, **characterized in that** a pre-connected collection container (60) is fastened to the catheter (10).

10. The catheter application system according to at least one of the claims 1 to 9, **characterized in that** the insertion sleeve (110) has a sleeve length between 1.55 and 2 cm.

11. The catheter application system according to at least one of the claims 1 to 10, **characterized in that** a locking device is provided within the insertion aid, said locking device allowing displacement of the catheter through the insertion aid in only the insertion direction.

## Revendications

1. Système d'application de cathéter comprenant :
un cathéter (10) apte à être appliqué à demeure pour le drainage de la vessie, qui présente une pointe de cathéter (11) et un ballonnet gonflable disposé dans la zone de la pointe de cathéter (11), le ballonnet, à l'état gonflé, ayant pour effet de bloquer la pointe de cathéter (11), de préférence dans la vessie du patient ;
une gaine creuse (20) dans laquelle le cathéter est disposé en glissement, et un auxiliaire d'introduction (100) à l'extrémité distale de la gaine, prévu pour guider le cathéter (10) à travers cette dernière, l'auxiliaire d'introduction présentant des parties d'auxiliaire d'introduction, plus précisément
un manchon d'introduction (110) comprenant un élément de fermeture (115) qui peut être ouvert pour le guidage du cathéter (10), et
un élément (130) faisant office de butée s'étendant à l'extérieur de la gaine, pour délimiter une introduction du manchon d'introduction (10) dans un orifice corporel, **caractérisé en ce que** l'auxiliaire d'introduction présente un bouchon de protection amovible (140) par dessus le manchon d'introduction (110), et **en ce que** le manchon d'introduction (110) et/ou l'élément (130) faisant office de butée sont réalisés sous la forme de parties d'auxiliaire d'introduction séparables de telle sorte qu'ils peuvent se séparer du cathéter (10) après le passage du cathéter (10), et **en ce que** l'élément faisant office de butée présente des segments de préhension, les segments de préhension étant réalisés en un matériau flexible déformable, possédant de préférence des propriétés élastiques.

2. Système d'application de cathéter selon la revendication 1, **caractérisé en ce que** l'élément de fermeture (115) présente une perforation de telle sorte que l'ouverture, lors du passage du cathéter (10) est rendue possible en perçant la perforation, ou bien l'élément de fermeture (115) présente une fente en croix, de telle sorte que l'ouverture lors du passage du cathéter (10) est rendue possible en perçant la fente en croix.

3. Système d'application de cathéter selon au moins une des revendications 1 à 2, **caractérisé en ce que** les parties de l'auxiliaire d'introduction réalisées de façon à pouvoir être séparées sont constituées respectivement par au moins deux segments (111, 112, 131, 132) qui, à l'état assemblé, renferment le cathéter réalisé (10) à la manière d'un anneau.

4. Système d'application de cathéter selon la revendication 3, **caractérisé en ce que** les segments (111, 112, 131, 132) des parties de l'auxiliaire d'introduction réalisées de façon à pouvoir être séparées sont maintenus ensemble au moyen de zones destinées à la rupture (150), en particulier au moyen de perforation et/ou d'une bande adhésive et/ou d'une bande en crêpe et/ou d'éléments d'encliquetage.

5. Système d'application de cathéter selon au moins une des revendications 1 à 4, **caractérisé en ce que** l'auxiliaire d'introduction, en particulier l'élément (130) faisant office de butée est réalisé sous la forme d'un élément de la dimension d'une poignée, une ouverture de passage unilatérale ou bilatérale (135) étant de préférence pratiquée dans l'élément.

6. Système d'application de cathéter selon au moins une des revendications 1 à 5, **caractérisé en ce que** le cathéter (10) présente une surface externe hydrophobe/oléophobe et/ou, sur sa surface externe et/ou interne, un revêtement antimicrobien, de préférence un revêtement à base d'argent.

7. Système d'application de cathéter selon au moins une des revendications 1 à 6, **caractérisé en ce que** le capuchon de protection (140) et/ou le manchon d'introduction (110) sont respectivement remplis d'un liquide et/ou **en ce qu'**un réservoir pour liquide (41) rempli d'un liquide est disposé dans l'auxiliaire d'introduction (10) pour pouvoir être transpercé par le cathéter (10) lors de l'application de ce dernier.

8. Système d'application de cathéter selon la revendication 7, **caractérisé en ce que,** en ce qui concerne les liquides, il s'agit d'un gel de glissement et/ou d'eau et/ou d'une solution saline physiologique.

9. Système d'application de cathéter selon au moins une des revendications 1 à 8, **caractérisé en ce qu'**un récipient de réception préconnecté est fixé au cathéter (10).

10. Système d'application de cathéter selon au moins une des revendications 1 à 9, **caractérisé en ce que** le manchon d'introduction (10) présente une longueur de manchon entre 1,55 et 2 cm.

11. Système d'application de cathéter selon au moins une des revendications 1 à 10, **caractérisé en ce qu'**on prévoit, au sein de l'auxiliaire d'introduction, un dispositif de blocage qui autorise le déplacement du cathéter via l'auxiliaire d'introduction, uniquement dans la direction d'introduction.
